# EUROPEAN PATENT SPECIFICATION

(11) **EP 0 800 813 B1**
(45) Date of publication and mention of the grant of the patent: **30.01.2002**
(21) Application number: 96830196.0
(22) Date of filing: 09.04.1996
(51) Int. Cl.: A61K 7/00, A61K 7/42

(54) **Cosmetic sunscreening compositions**
Kosmetische Sonnenschutzmitteln
Compositions cosmétiques formant écran solaire

(43) Date of publication of application: 15.10.1997
(73) Proprietor: 3V SIGMA S.p.A, 20121 Milano (IT)
(72) Inventor: Raspanti, Giuseppe, 24121 Bergamo (IT); Malpede, Alverio, 24121 Bergamo (IT)
(74) Representative: Minoja, Fabrizio, Dr.

(56) References cited:
- EP-A- 0 517 104
- EP-A- 0 570 838
- EP-A- 0 689 828

## Description

The present invention relates to photostable cosmetic compositions containing sunscreens for radiations ranging from 280 to 400 nm and their use for the protection of the skin from UV radiations.

It is known that sun radiations ranging from 280 to 400 nm are noxious to human skin; in particular those having wavelength between 280 and 320 nm, the so-called UV-B radiations, cause erythema and sunburns, whose severity depends on the length of the exposition and on the skin type. It has been ascertained that also radiations ranging from 320 to 400 nm, so called UV-A, responsible of skin tanning, can provoke alterations and damages in the skin, which may not be disregarded, especially in case of sensible skins or in case of a continuous exposition to radiations.

It has been demonstrated that the UV-A radiation, other than damaging elastin and collagen, whereby skin ageing, can also induce a number of phototoxic and photoallergic reactions. Moreover UV-B noxious action can be enhanced by the presence of UV-A (Willis et al.: Journal of Investigative Dermatology vol. 59, 416, 1972).

For the protection from UV-B radiations some derivatives of cinnamic acid, 4-aminobenzoic acid, benzylydencamphor and benzophenone, are known and used for the preparation of cosmetic compositions. These compounds, other than a more or less sufficient efficacy, also are endowed with an acceptable photostability.

On the contrary, sufficiently effective products are not yet available for the protection from UV-A, even if in the patent literature different compounds have been provided; but their practical outcome has not given positive results.

To date UV-A sunscreens (absorbers) used in pratice are limited to benzophenone derivatives and to some dibenzoylmethane derivatives.

Among the benzophenone derivatives 2-hydroxy-4-methoxybenzophenone is the more used, whose maximum absorption, at about 325 nm, is too low to be able to provide a sufficient protection, moreover, due to its poor solubility, its use in compositions is difficult.

Among the dibenzoylmethane derivatives, 4-methoxy-4'-ter-butyl-dibenzoylmethane and 4-isopropyldibenzoylmethane are those commercially known.

These suncreens have a good absorption (E¹₁ about 1100) at about 360 nm and an acceptable solubility.

However, their use is difficult since they have the severe drawback of not being sufficiently photostable (Ing. J. Cosm. Science 10, 53, 1988), whereby the compositions containing these compounds can not guarantee a sufficient protection from UV-A because the sunscreen is fastly degraded by the radiation itself. In order to avoid this fast degradation and give a certain photostabilty to these UV-A filters, in patents GB 2 198 944, PCT WO 91/11989 and PCT WO 94/04131 particular combinations of UV-B filters belonging to the class of the benzylydencamphor and diphenylcyanoacrylate and derivatives of dibenzoylmethane are proposed.

Nevertheless, the results are not yet satisfying, also because, to obtain a certain photostability of the dibenzoylmethane derivatives, excessive amounts of UV-B filters must be added.

Therefore, the problem of the stabilization of sunscreens, in particular the derivatives of dibenzoylmethane in cosmetic compositions is still urgent. Moreover, the problem of cosmetic treatment of the skin exsposed to sunlight, as to allow a pleasant tanning without incurring in sunburns, is still felt.

It has surprisingly been found that a good photostability of the derivatives of dibenzoylmethane can be obtained by combining a derivative of dibenzoylmethane of formula (I) 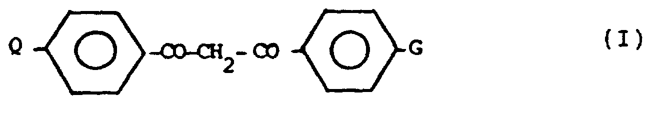
wherein Q is hydrogen, alkoxy with C₁-C₄ alkyl group and
G is C₁-C₈ straight o branched alkyl, with one of the compounds of formula (II), having high absorbption in the UV-B region, described in the US Patent 5,346,691 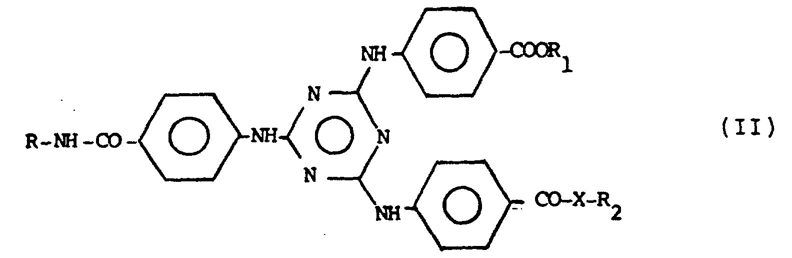 wherein
R is C₁-C₈ straight o branched alkyl, C₅-C₁₂ cycloalkyl optionally substituted with one or more C₁-C₄ alkyl;
X is oxygen or the -NH- group;
R₁ has the same meanings of R or is hydrogen, an alkali metal, an ammonium group or a group of formula (III): 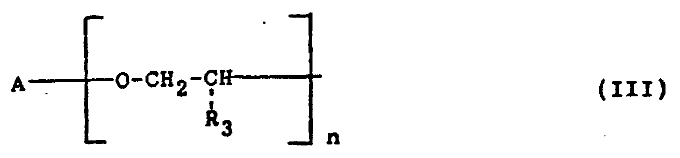
wherein A is C₁-C₈ straight o branched alkyl, C₅-C₈ cycloalkyl, aryl optionally substituted with one or more C₁-C₄ alkyl, R₃ is hydrogen or methyl and n is an integer from 1 to 10;
R₂ has the same meanings of R when X is the -NH- group or has the same meaning of R₁ when X is oxygen.

Preferred compounds of formula (I) are those in which Q is methoxy and G is ter-butyl, or Q is hydrogen and G is isopropyl.

Preferred compounds of formula (II) are those in which R=CH₃, R₁=R₂=C₄H₉-CH(C₂H₅)-CH₂-, X=O; R=(CH₃)₃C-CH₂-C(CH₃)₂-, R₁=R₂=C₄H₉CH(C₂H₅)-CH₂-, X=O; R=R₂=(CH₃)₂CH-, R₁=C₁₄H₂₉-, X=NH; R=(CH₃)₃C-, R₁=R₂=C₄H₉-CH(C₂H₅)-CH₂-, X=O.

The weight ratio between one or more compounds of formula (II) and one or more compounds of formula (I) can be up to 6.

The derivatives of dibenzoylmethane of formula (I) are described in US patents 4,387,089 and 4,489,057. The commercial compounds are Parsol^{(R)}1789 (4-methoxy-4'-ter-butyldibenzoylmethane) by Givaudan and Eusolex^{(R)}8020 (4-isopropyldibenzoylmethane) by Merck. By means of their combination with a UV-B filter of formula (II) cosmetic compositions are obtained absorbing radiations with wavelength ranging from 290 to 380 nm and then with protective action both in UV-B and in UV-A, but especially UV-A filter enjoys an optimal photochemical stabilization. It is therefore possible to prepare cosmetic compositions suitable to guarantee a continuous protection of the skin also during a prolonged exposition to sunlight, thereby avoiding frequent and repeated applications necessary for an effective protection.

According to the present invention, the photostable cosmetic compositions suitable for the protection of the skin from UV radiations comprise a cosmetic substrate containing, with respect to the weight of the composition, from 1 to 5% of one or more dibenzoylmethane derivatives of formula (I) and from 1 to 6% of a compound of formula (II), with the proviso that the weight ratio between the compounds of formula (II) and those of formula (I) be at least 1.

The compositions according to the present invention absorb UV radiations in the region comprised from 290 to 380 nm, and can be effectively used for the protection of the skin from UV-A and UV-B sun radiations.

The compositions according to the present invention are also useful for the treatment, therefore the protection, of hairs or make-up in decorative cosmetics.

According to the present invention the cosmetic compositions can be solutions, lotions, emulsions of the water-in-oil or oil-in-water type; or can also be in the form of gels, lipsticks, aerosol.

The compositions according to the present invention are prepared by formulating usually used ingredients, such as for example oils, fats, emollients, hydrating agents, moisturizing agents, softening agents, preservatives, surfactants, thickening agents, antifoam, perfumes, pigments, dyes and other else such as alcohols, polyols, electrolytes, silicone derivatives.

The most commonly used solvents are triglycerides of caprinic or caprilic acid, for example castor oil, esters of fatty acids with isopropanol, propylene glycol, glycerin, propylene glycole-monomethyl or monoethyl or monobutyl ether.

The cosmetic compositions according to the present invention other than UV-absorbers of formula (I) and (II) can contain also other sunscreens normally used in cosmetic, such as for example: 3-(4-methylbenzylydene)-camphor; 2-ethylhexyl-(4-dimethylamino)benzoate, 2-ethylhexyl-4-methoxy-cinnamate, menthyl salicilate 2-ethylhexyl-2-cyano-3,3-diphenylacrylate, 2-hydroxy-4-methoxy-benzophenone, 2,4,6-trianilino-(p-carbo-2-ethylhexyloxy)-1,3,5-triazine, salts of 2-phenyl-benzimidazol-5-sulfonic acid or of 2-hydroxy-4-methoxy-benzophenone-5-sulfonic acid.

The present invention also comprises the protection of the cosmetic composition itself from UV radiation by means of the use of the combinations of compounds of formula (II) with the derivatives of dibenzoylmethane of formula (I). In this case there are compositions whose components can undergo degradation or unwanted colourations caused by the light, such as for example shampoes and hair lacquers, lotions for the hair dressing, compositions for hair dyeing, compositions for make-up, such as nail lacquers, foundation, lipstick. Preferred cosmetic formulations are those for the protection of the skin from sun radiations.

The cosmetic compositions to be protected according to the present invention can contain 1-5% of one or more compounds of formula (I) and 1-6% of compounds of formula (II), by weight with respect to the total of the composition.

According to the present invention the cosmetic compositions can contain also inorganic pigments, usually used in cosmetics, such as for example titanium, zinc, silicon or aluminium oxides.

The following examples further illustrate the invention.

### Example 1

| Lotion | |
|---|---|
| Compound A | 3.0 |
| 4-Methoxy-4'-terbutylbenzoylmethane (Persol^{(R)}1789) | 2.0 g |
| Isopropylmirystate q.b. | 100.0 g. |

The components are mixed and homogeneized by means of stirring at 30-40°C for 10 minutes.

Compound A = Compound of formula (II)

R=CH₃-; R₁=R₂=C₄H₉-CH(C₂H₅)-CH₂-; X=O

1 g of lotion is diluted in 1000 ml of ethanol; 10 ml of the obtained solution are irradiated by means of a sun simulator for 10 hours.

The content of UV-A filter Parsol^{(R)}1789 is determined by means of HPLC before and after irradiation. The loss of Parsol^{(R)}1789 is negligible when compared with the loss that occurs in a lotion containing only Parsol^{(R)}1789.

### Example 2

| Sun milk | |
|---|---|
| Triglyceride of fatty acid | 20.0 g |
| Cetyl alcohol | 2.0 g |
| Cetylstearyl alcohol | 2.0 g |
| Lanoline | 4.0 g |
| Siliconic oil | 0.4 g |
| 4-Methoxy-4'-ter-butyldibenzoylmethane | 3 g |
| Compound B | 3.5 g |
| Abiol^{(R)} (preservative by 3V-SIGMA) | 0.2 g |
| Synthalen^{(R)}M (crosslinked polyacrilic acid by 3V SIGMA) | 0.1 g |
| Triethanolamine | 0.15 g |
| Perfume | 0.3 g |
| Distilled water q.s.to | 100.0 g |

The fatty phase is warmed to 80-90°C, the sunscreen is added, then the mixture is added to water containing the hydrosoluble compounds, heated to 80-90°C. Stirring is continued under warming for 15-20 minutes. The mixture is slowly cooled and perfume is added.

Compound B = Compound of formula (II)

R=(CH₃)₃C-CH₂-C(CH₃)₂-; R₁=R₂=C₄H₉CH(C₂H₅)-CH₂-; X=O

### Example 3

| Day cream | |
|---|---|
| Triglyceride of (C ₈ -C ₁₈ ) acid | 29.0 g |
| Glycerin monostearate | 7.0 g |
| Stearic acid | 2.0 g |
| Lanoline | 4.0 g |
| Preservative | 0.2 g |
| Compound C | 2.5 g |
| 4-Isopropyldibenzoylmethane (Eusolex ^{(R)} 8020) | 1.5 g |
| Propylene glycole | 2.5 g |
| Triethanolamine | 0.5 g |
| Perfume | 0.3 g |
| Distilled water q.s. to | 100.0 g. |

The preparation is carried out as described in Example 2.

Compound C = Compound of formula (II)

R=R₂=(CH₃)₂CH-; R₁=C₁₄H₂₉-; X=NH

### Example 4

| Sun cream | |
|---|---|
| Benzoate of C₁₂-C₁₅ alcohols (Finisol^{(R)}IN, Witco) | 5.0 g |
| Cetylstearyl alcohol | 3.0 g |
| Glycerin mono and distearate | 4.0 g |
| Polyglycole (Arlacel^{(R)}165 ICI) | 2,0 g |
| Mirystic alcohol with 3 moles of propylene oxide (Witconol^{(R)}APM-Witco) | 21.0 g |
| Compound D | 5.0 g |
| 4-Methoxy-4'-ter-butyl-dibenzoylmethane (Parsol^{(R)}1789) | 3.0 g |
| Distilled water q.s. to | 100.0 g. |

The procedure is the same as described in Example 2.

Compound D = Compound of formula (II)

R=(CH₃)₃C-; R₁=R₂=C₄H₉-CH(C₂H₅)-CH₂-; X=O

### Example 5

| Alcoholic gel | |
|---|---|
| Propylene glycole | 25,0 g |
| Ethyl alcohol 96% | 25,0 g |
| Synthalen ^{(R)} M (crosslinked polyacrilic acid by 3V SIGMA) | 0,6 g |
| Compound A | 1,5 g |
| 4-Methoxy-4'-ter-butyl-dibenzoylmethane (Parsol ^{(R)} 1789) | 1,0 g |
| Triethanolamine | 0,3 g |
| Preservative | 0,3 g |
| Perfume | 0,3 g |
| Distilled water q.s. to | 100,0 g. |

Synthalen^{(R)}M is dispersed into water, then triethanolamine is added and the mixture of propylene glycole and ethanol, wherein compound A and Parsol^{(R)}1789 were previously dissolved, is finally added.

By repeating the same test described in Example 1 for Examples 2-5 the loss of Parsol^{(R)}1789 is found to be always lower than the one that it occurs when Parsol^{(R)}1789 alone is used.

## Claims (Claims for the following Contracting State(s): BE, DE, ES, FR, GB, IT, NL.)

1. A cosmetic composition comprising a cosmetic substrate containing from 1 to 5% by weight of one or more derivatives of dibenzoylmethane of formula (I) 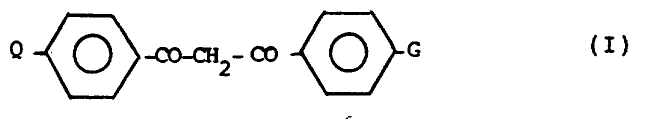 wherein Q is hydrogen or alkoxy with C₁-C₄ alkyl group and G is C₁-C₄ straight or branched alkyl and from 1 to 6% by weight of one or more compounds of formula (II) 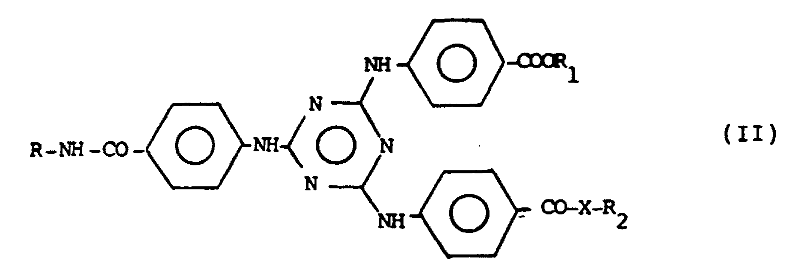 wherein
R is C₁-C₈ straight or branched alkyl, C₅-C₁₂ cycloalkyl optionally substituted with one or more C₁-C₄ alkyl;
X is oxygen or the -NH- group;
R₁ has the same meaning of R or is hydrogen, an alkali metal, an ammonium group or a group of formula (III): 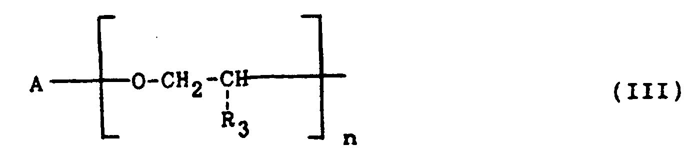 wherein A is C₁-C₈ straight or branched alkyl, C₅-C₈ cycloalkyl, aryl optionally substituted with one or more C₁-C₄ alkyl, R₃ is hydrogen or methyl and n is an integer from 1 to 10;
R₂ has the same meanings of R when X is the -NH- group or R₂ has the same meaning of R₁ when X is oxygen; with the proviso that the weight ratio between the compounds of formula (II) and those of formula (I) is at least 1.

2. Cosmetic compositions according to claim 1 in which in formula (I) Q is methoxy and G is ter-butyl.

3. Cosmetic compositions according to claim 1 in which in formula (I) Q is hydrogen and G is isopropyl.

4. Cosmetic compositions according to claims 1-3, in which the weight ratio between one or more compounds of formula (II) and one or more compounds of formula (I) is maximum 6.

5. Compositions according to claims 1-4 containing also zinc oxide.

6. Compositions according to claims 1-4, containing also titanium oxide.

7. Compositions according to anyone of claims 1-6, containing also other sunscreens selected from the group consisting of 3-(4-methylbenzylydene)-camphor; 2-ethylhexyl-(4-dimethylamino)benzoate, 2-ethylhexyl-4-methoxy-cinnamate, menthyl salicilate, 2-ethylhexyl-2-cyano-3,3-diphenylacrylate, 2-hydroxy-4-methoxy-benzophenone, 2,4,6-trianilino-(p-carbo-2-ethylhexyloxy)-1,3,5-triazine, salts of 2-phenyl-benzimidazol-5-sulfonic acid or of 2-hydroxy-4-methoxy-benzophenone-5-sulfonic acid.

8. Compositions according to anyone of claims 1-7, containing as coadjuvants one or more components selected from thickening agents, emollients, hydrating agents, preservatives, perfume.

9. Use of the cosmetic compositions according to anyone of claims 1-8, for the non-therapeutic treatment of human skin when exposed to sunlight.

10. Process for the protection of cosmetic compositions from UV radiations comprising the addition to the cosmetic substrate of from 1 to 5% of a compound of formula (I) 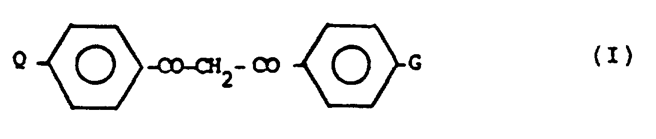 in which Q is hydrogen or alkoxy with C₁-C₄ alkyl group and G is C₁-C₄ straight or branched alkyl and from 1 to 6% of a compound of formula (II) 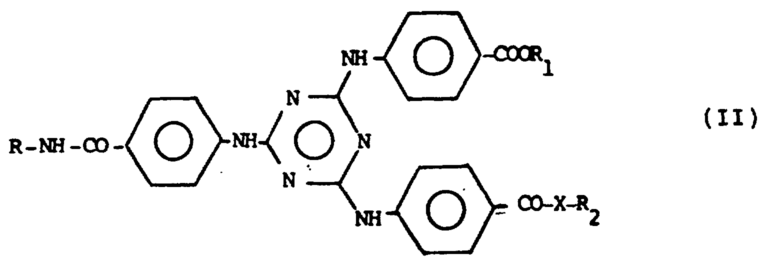 in which
R is C₁-C₈ straight or branched alkyl, C₅-C₁₂ cycloalkyl optionally substituted with one or more C₁-C₄ alkyl;
X is oxygen or the -NH- group;
R₁ has the same meanings of R or is hydrogen, an alkali metal, an ammonium group or a group of formula (III): 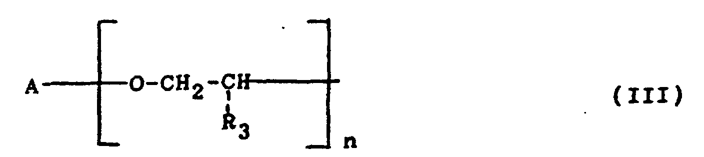
in which A is C₁-C₈ straight or branched alkyl, C₅-C₈ cycloalkyl, aryl optionally substituted with one or more C₁-C₄ alkyl, R₃ is hydrogen or methyl and n can be an integer from 1 to 10;
R₂ has the same meanings of R when X is the -NH- group or R₂ has the same meaning of R₁ when X is oxygen.

11. Photostabilizing mixture consisting of at least one dibenzoylmethane derivative of formula (I) 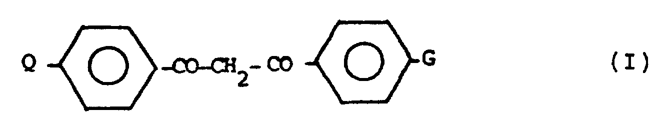 in which Q is hydrogen or alkoxy with C₁-C₄ alkyl group and G is C₁-C₄ straight or branched alkyl and at least one compound of formula (II) 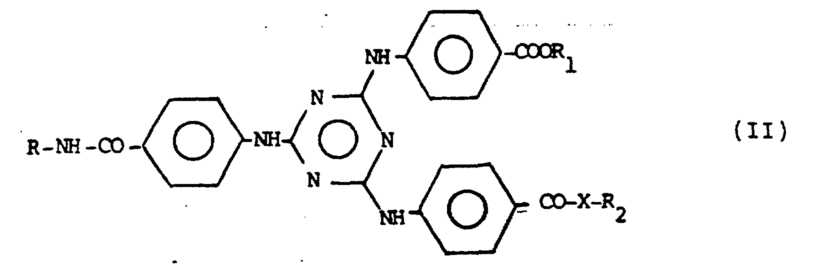 in which
R is C₁-C₈ straight or branched alkyl, C₅-C₁₂ cycloalkyl optionally substituted with one or more C₁-C₄ alkyl;
X is oxygen or the -NH- group;
R₁ has the same meanings of R or is hydrogen, an alkali metal, an ammonium group or a group of formula (III): 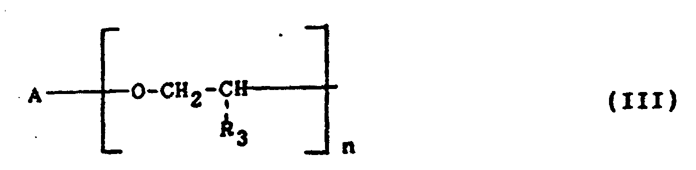
in which A is C₁-C₈ straight or branched alkyl, C₅-C₈ cycloalkyl, aryl optionally substituted with one or more C₁-C₄ alkyl, R₃ is hydrogen or methyl and n can be an integer from 1 to 10;
R₂ has the same meanings of R when X is the -NH- group or R₂ has the same meaning of R₁ when X is oxygen; being the weight ratio between said compounds (II) and said compounds (I) ranging from 1 to 6.

12. Use of the mixture of the claim 11 for the preparation of a dermatological composition for the protection of the human skin from ultraviolet radiations.

## Patentansprüche (Patentansprüche für folgende(n) Vertragsstaat(en): BE, DE, ES, FR, GB, IT, NL)

1. Kosmetische Zubereitung, umfassend ein kosmetisches Substrat, enthaltend 1 bis 5 Gew.-% eines oder mehrerer Derivate des Dibenzoylmethans der Formel (I) 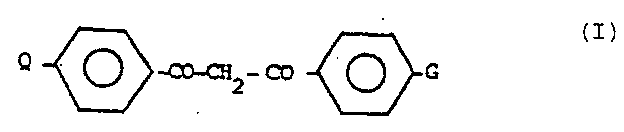 worin Q Wasserstoff oder Alkoxy mit C₁-C₄-Alkylgruppe bedeutet und G geradkettiges oder verzweigtes C₁-C₄-Alkyl bedeutet, und 1 bis 6 Gew.-% einer oder mehrerer Verbindungen der Formel (II) 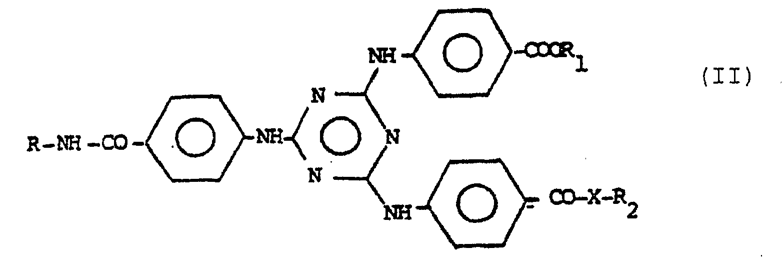 worin
R geradkettiges oder verzweigtes C₁-C₈-Alkyl, C₅-C₁₂-Cycloalkyl, gegebenenfalls substituiert mit einem oder mehreren C₁-C₄-Alkyl bedeutet;
X Sauerstoff oder die Gruppe -NH- bedeutet;
R₁ die gleiche Bedeutung wie R besitzt oder Wasserstoff, ein Alkalimetall, eine Ammoniumgruppe oder eine Gruppe der Formel (III): 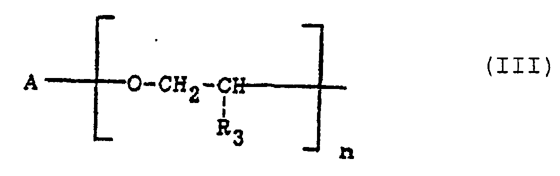 worin A geradkettiges oder verzweigtes C₁-C₈-Alkyl, C₅-C₈-Cycloalkyl, Aryl, gegebenenfalls substituiert mit einem oder mehreren C₁-C₄-Alkyl, bedeutet, R³ Wasserstoff oder Methyl bedeutet und n eine ganze Zahl von 1 bis 10 ist, bedeutet;
R₂ die gleichen Bedeutungen wie R besitzt, wenn X die Gruppe -NH- bedeutet, oder R₂ die gleichen Bedeutungen wie R₁ besitzt, wenn X Sauerstoff bedeutet;
mit der Maßgabe, dass das Gewichtsverhältnis zwischen den Verbindungen der Formel (II) und solchen der Formel (I) mindestens 1 beträgt.

2. Kosmetische Zubereitungen nach Anspruch 1, wobei in der Formel (I) Q Methoxy und G ter-Butyl bedeuten.

3. Kosmetische Zubereitungen nach Anspruch 1, wobei in der Formel (I) Q Wasserstoff und G Isopropyl bedeuten.

4. Kosmetische Zubereitungen nach einem der Ansprüche 1 bis 3, wobei das Gewichtsverhältnis zwischen einer oder mehreren Verbindungen der Formel (II) und einer oder mehreren Verbindungen der Formel (I) maximal 6 beträgt.

5. Zubereitungen nach einem der Ansprüche 1 bis 4, die außerdem Zinkoxid enthalten.

6. Zubereitungen nach einem der Ansprüche 1 bis 4, die außerdem Titanoxid enthalten.

7. Zubereitungen nach einem der Ansprüche 1 bis 6, enthaltend ebenfalls andere Sonnenschutzmittel, ausgewählt aus der Gruppe bestehend aus 3-(4-Methylbenzyliden)-campher; 2-Ethylhexyl-(4-dimethylamino)benzoat, 2-Ethylhexyl-4-methoxycinnamat, Menthylsalicylat, 2-Ethylhexyl-2-cyano-3,3-diphenylacrylat, 2-Hydroxy-4-methoxybenzophenon, 2,4,6-Trianilino-(p-carbo-2-ethylhexyloxy)-1,3,5-triazin, Salze von 2-Phenylbenzimidazol-5-sulfonsäure oder von 2-Hydroxy-4-methoxybenzophenon-5-sulfonsäure.

8. Zubereitungen nach einem der Ansprüche 1 bis 7, enthaltend als Coadjuvantien eine oder mehrere Komponenten, ausgewählt aus Verdickungsmitteln, erweichenden Mitteln, Hydratisierungsmitteln, Konservierungsmitteln und Parfums.

9. Verwendung der kosmetischen Zubereitungen nach einem der Ansprüche 1 bis 8 für die nicht-therapeutische Behandlung der humanen Haut, wenn sie Sonnenlicht ausgesetzt ist.

10. Verfahren zum Schutz von kosmetischen Zubereitungen vor UV-Bestrahlungen, umfassend die Zugabe zu dem kosmetischen Substrat von 1 bis 5 % einer Verbindung der Formel (I) 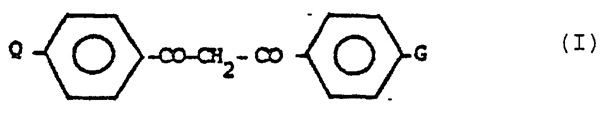 worin Q Wasserstoff oder Alkoxy mit C₁-C₄-Alkylgruppe bedeutet und G geradkettiges oder verzweigtes C₁-C₄-Alkyl bedeutet und von 1 bis 6 % einer oder mehrerer Verbindungen der Formel (II) 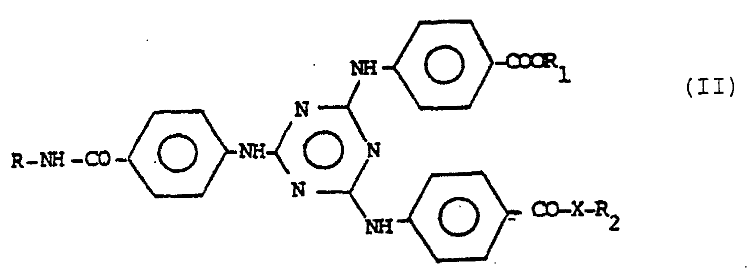 worin
R geradkettiges oder verzweigtes C₁-C₈-Alkyl, C₅-C₁₂-Cycloalkyl, gegebenenfalls substituiert mit einem oder mehreren C₁-C₄-Alkyl bedeutet;
X Sauerstoff oder die Gruppe -NH- bedeutet;
R₁ die gleiche Bedeutung wie R besitzt oder Wasserstoff, ein Alkalimetall, eine Ammoniumgruppe oder eine Gruppe der Formel (III): 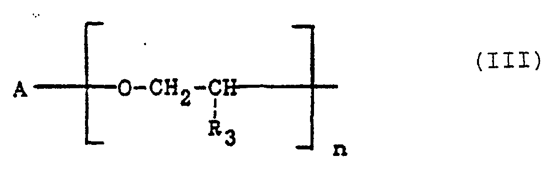 worin A geradkettiges oder verzweigtes C₁-C₈-Alkyl, C₅-C₈-Cycloalkyl, Aryl, gegebenenfalls substituiert mit einem oder mehreren C₁-C₄-Alkyl, bedeutet, R₃ Wasserstoff oder Methyl bedeutet und n eine ganze Zahl von 1 bis 10 ist, bedeutet;
R₂ die gleichen Bedeutungen wie R besitzt, wenn X die Gruppe -NH- bedeutet, oder R₂ die gleichen Bedeutungen wie R₁ besitzt, wenn X Sauerstoff bedeutet.

11. Photostabilisierendes Gemisch, bestehend aus mindestens einem Dibenzoylmethanderivat der Formel (I) 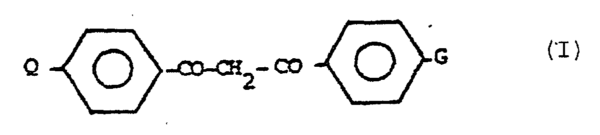 worin Q Wasserstoff oder Alkoxy bedeutet, mit C₁-C₄-Alkylgruppe und G geradkettiges oder verzweigtes C₁-C₄-Alkyl bedeutet, und mindestens eine Verbindung der Formel (II) 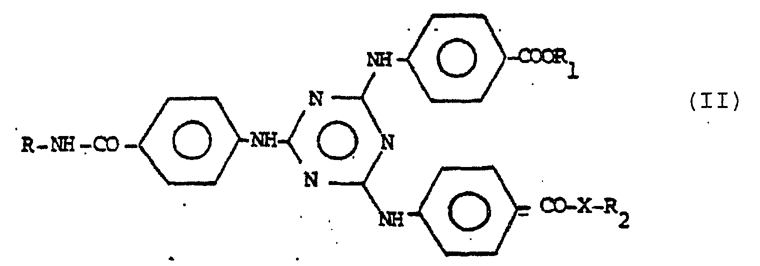 worin
R geradkettiges oder verzweigtes C₁-C₈-Alkyl, C₅-C₁₂-Cycloalkyl, gegebenenfalls substituiert mit einem oder mehreren C₁-C₄-Alkyl, bedeutet;
X Sauerstoff oder die Gruppe -NH- bedeutet;
R₁ die gleiche Bedeutung wie R besitzt oder Wasserstoff, ein Alkalimetall, eine Ammoniumgruppe oder eine Gruppe der Formel (III): 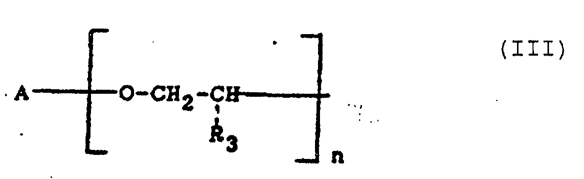 worin A geradkettiges oder verzweigtes C₁-C₈-Alkyl, C₅-C₈-Cycloalkyl, Aryl, gegebenenfalls substituiert mit einem oder mehreren C₁-C₄-Alkyl, bedeutet, R₃ Wasserstoff oder Methyl bedeutet und n eine ganze Zahl von 1 bis 10 sein kann, bedeutet;
R₂ die gleichen Bedeutungen wie R besitzt, wenn X die Gruppe -NH- bedeutet, oder R₂ die gleiche Bedeutung wie R₁ besitzt, wenn X Sauerstoff bedeutet; wobei das Gewichtsverhältnis zwischen den Verbindungen (II) und den Verbindungen (I) im Bereich von 1 bis 6 liegt.

12. Verwendung des Gemisches von Anspruch 11 zur Herstellung einer dermatologischen Zubereitung für den Schutz der humanen Haut vor ultravioletten Bestrahlungen.

## Revendications (Revendications pour l'(les) Etat(s) contractant(s) suivant(s): BE, DE, ES, FR, GB, IT, NL.)

1. Composition cosmétique comprenant un substrat cosmétique contenant de 1. à 5 % en poids d'un ou plusieurs dérivés de dibenzoylméthane de formule (I) 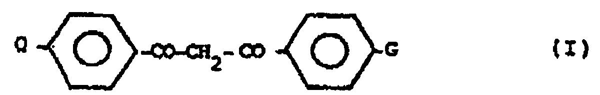 dans laquelle Q est de l'hydrogène ou un groupe alcoxy avec un groupe alkyle en C₁ à C₄ et G est un groupe alkyle en C₁ à C₄ linéaire ou ramifié, et de 1 à 6 % en poids d'un ou plusieurs composés de formule (II) 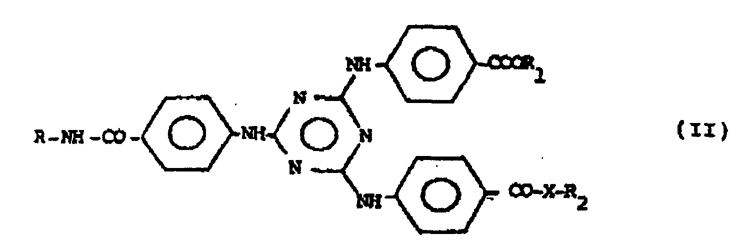 dans laquelle
R est un groupe alkyle en C₁ à C₈ linéaire ou ramifié, cycloalkyle en C₅ à C₁₂ éventuellement substitué avec un ou plusieurs groupes alkyles en C₁ à C₄ ;
X est de l'oxygène ou le groupe -NH- ;
R₁ a la même signification que R ou est de l'hydrogène, un métal alcalin, un groupe ammonium ou un groupe de formule (III) : 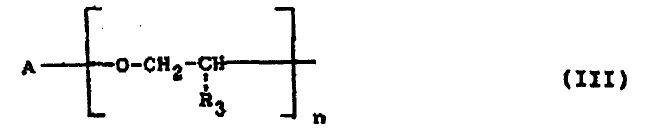 dans laquelle A est un groupe alkyle en C₁ à C₈ linéaire ou ramifié, cycloalkyle en C₅ à C₈, aryle éventuellement substitué avec un ou plusieurs groupes alkyles en C₁ à C₄, R₃ est de l'hydrogène ou un groupe méthyle et n est un entier de 1 à 10 ;
R₂ a les mêmes significations que R lorsque X est le groupe -NH- ou R₂ a la même signification que R₁ lorsque X est de l'oxygène ; étant entendu que le rapport pondéral entre les composés de formule (II) et ceux de formule (I) est au moins 1.

2. Compositions cosmétiques selon la revendication 1, dans lesquelles dans la formule (I) Q est un groupe méthoxy et G est un groupe ter-butyle.

3. Compositions cosmétiques selon la revendication 1, dans lesquelles dans la formule (I) Q est de l'hydrogène et G est un groupe isopropyle.

4. Compositions cosmétiques selon les revendications 1-3, dans lesquelles le rapport pondéral entre un ou plusieurs composés de formule (II) et un ou plusieurs composés de formule (I) est au maximum 6.

5. Compositions selon les revendications 1-4, contenant aussi de l'oxyde de zinc.

6. Compositions selon les revendications 1-4, contenant aussi de l'oxyde de titane.

7. Compositions selon l'une quelconque des revendications 1-6, contenant aussi d'autres écrans solaires choisis dans le groupe constitué par le 3-(4-méthylbenzylydène)camphre ; le (4-diméthylamino)benzoate de 2-éthylhexyle, le 4- méthoxy-cinnamate de 2-éthylhexyle, le salicilate de menthyle, le 2-cyano-3,3-diphénylacrylate de 2-éthylhexyle, la 2-hydroxy-4-méthoxy-benzophénone, la 2,4,6-trianilino-(p-carbo-2-éthylhexyloxy)-1,3,5-triazine, les sels de l'acide 2-phényl-benzimidazol-5-sulfonique ou de l'acide 2-hydroxy-4-méthoxy-benzophénone-5-sulfonique.

8. Compositions selon l'une quelconque des revendications 1-7, contenant en tant que co-adjuvants un ou plusieurs constituants choisis parmi les agents épaississants, les émollients, les agents hydratants, les conservateurs, les parfums.

9. Utilisation des compositions cosmétiques selon l'une quelconque des revendications 1-8, pour le traitement non thérapeutique de peau humaine lorsqu'elle est exposée à de la lumière solaire.

10. Procédé pour protéger des compositions cosmétiques de radiations UV, comprenant l'addition au substrat cosmétique de 1 à 5 % d'un composé de formule (I) 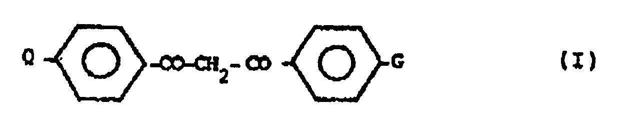 dans laquelle Q est de l'hydrogène ou un groupe alcoxy avec un groupe alkyle en C₁ à C₄ et G est un groupe alkyle en C₁ à C₄ linéaire ou ramifié, et de 1 à 6 % en poids d'un composé de formule (II) 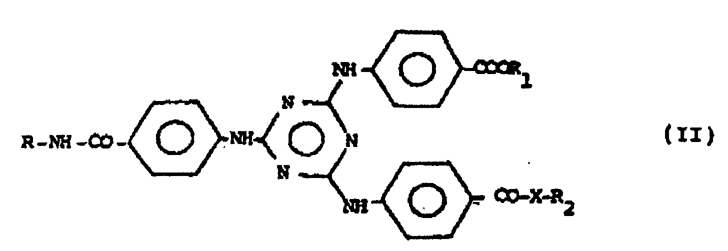 dans laquelle
R est un groupe alkyle en C₁ à C₈ linéaire ou ramifié, cycloalkyle en C₅ à C₁₂ éventuellement substitué avec un ou plusieurs groupes alkyles en C₁ à C₄ ;
X est de l'oxygène ou le groupe -NH- ;
R₁ a la même signification que R ou est de l'hydrogène, un métal alcalin, un groupe ammonium ou un groupe de formule (III) : 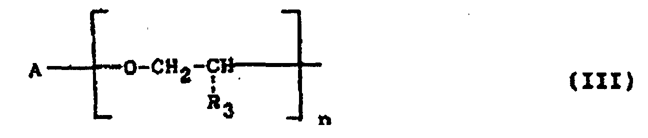 dans laquelle A est un groupe alkyle en C₁ à C₈ linéaire ou ramifié, cycloalkyle en C₅ à C₈, aryle éventuellement substitué avec un ou plusieurs groupes alkyles en C₁ à C₄, R₃ est de l'hydrogène ou un groupe méthyle et n peut être un entier de 1 à 10 ;
R₂ a les mêmes significations que R lorsque X est le groupe -NH- ou R₂ a la même signification que R₁ lorsque X est de l'oxygène.

11. Mélange photostabilisant consistant en au moins un dérivé de dibenzoylméthane de formule (I) 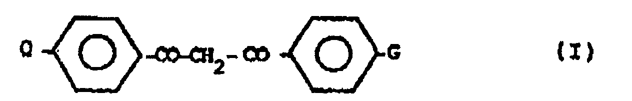 dans laquelle Q est de l'hydrogène ou un groupe alcoxy avec un groupe alkyle en C₁ à C₄ et G est un groupe alkyle en C₁ à C₄ linéaire ou ramifié, et au moins un composé de formule (II) 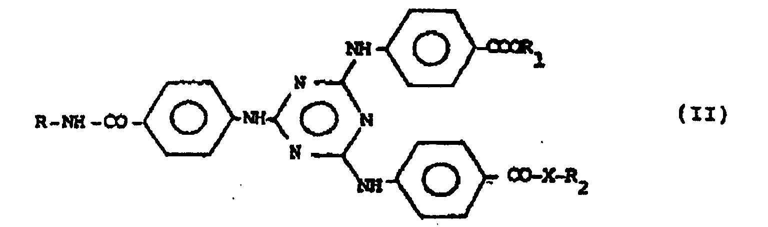 dans laquelle
R est un groupe alkyle en C₁ à C₈ linéaire ou ramifié, cycloalkyle en C₅ à C₁₂ éventuellement substitué avec un ou plusieurs groupes alkyles en C₁ à C₄ ;
X est de l'oxygène ou le groupe -NH- ;
R₁ a la même signification que R ou est de l'hydrogène, un métal alcalin, un groupe ammonium ou un groupe de formule (III) : 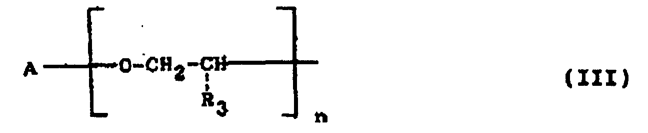 dans laquelle A est un groupe alkyle en C₁ à C₈ linéaire ou ramifié, cycloalkyle en C₅ à C₈, aryle éventuellement substitué avec un ou plusieurs groupes alkyles en C₁ à C₄, R₃ est de l'hydrogène ou un groupe méthyle et n peut être un entier de 1 à 10 ;
R₂ a les mêmes significations que R lorsque X est le groupe -NH- ou R₂ a la même signification que R₁ lorsque X est de l'oxygène ; étant entendu que le rapport pondéral entre lesdits composés (II) et lesdits composés (I) va de 1 à 6.

12. Utilisation du mélange selon la revendication 11 pour la préparation d'une composition dermatologique pour la protection de la peau humaine de radiations ultraviolettes.
